(19) Europäisches Patentamt
European Patent Office
Office européen des brevets

(11) Veröffentlichungsnummer : **0 282 749 B1**

(12) **EUROPÄISCHE PATENTSCHRIFT**

(45) Veröffentlichungstag der Patentschrift : **28.08.91 Patentblatt 91/35**

(51) Int. Cl.⁵ : **A61K 7/13**, C07C 229/58, C07C 229/60, C07C 309/28, C07C 309/40

(21) Anmeldenummer : **88102337.8**

(22) Anmeldetag : **18.02.88**

# (54) Haarfärbemittel mit direktziehenden Nitrodiphenylamin-Derivaten.

(30) Priorität : **26.02.87 DE 3706224**

(43) Veröffentlichungstag der Anmeldung : **21.09.88 Patentblatt 88/38**

(45) Bekanntmachung des Hinweises auf die Patenterteilung : **28.08.91 Patentblatt 91/35**

(84) Benannte Vertragsstaaten : **AT BE CH DE ES FR GB GR IT LI LU NL SE**

(56) Entgegenhaltungen :
**DE-B- 2 830 497**
**GB-A- 600 640**
**US-A- 2 611 785**
**US-A- 4 029 815**
**Römmps Chemie Lexicon, Achte Auflage, Franckh'sche Verlagshandlung Stuttgart, Seite 2821**

(73) Patentinhaber : **Henkel Kommanditgesellschaft auf Aktien**
**Postfach 1100 Henkelstrasse 67**
**W-4000 Düsseldorf-Holthausen (DE)**

(72) Erfinder : **Rose, David, Dr.**
**Holbeinweg 7**
**W-4010 Hilden (DE)**
Erfinder : **Lieske, Edgar**
**Hunsrückenstrasse 40**
**W-4000 Düsseldorf (DE)**
Erfinder : **Maak, Nobert, Dr.**
**Im Jagdfeld 41 a**
**W-4040 Neuss (DE)**

EP 0 282 749 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der Erfindung sind Haarfärbemittel mit einem Gehalt an direktziehenden Haarfarbstoffen. Solche Harrfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch einerseits eine begrenzte Wasserlöslichkeit, die zu Problemen bei der Formulierung der Haarfärbemittel führt, andererseits die unbefriedigende Waschechtheit, d. h. daß die Haarfärbungen nach mehrmaligem Haarwaschen stark ausbluten.

Römpps chemie Lexikon (Achte Auflage, 1985, Seite 2821) beschreibt, daß Nitrofarbstoffe mit saurem Charakter, die neben Nitrogruppen noch o-oder p-ständige Amino bzw. Hydroxylgruppen enthalten, schlechte waschechtheit aufweisen.

Weiterhin ist es wünschenswert, um modische Haarfärbungen zu erhalten, auch rote Farbtöne durch direktziehende Farbstoffe zu erzeugen. Dafür werden gewöhnlich 2-Nitro-p-phenylendiamin und dessen amino-substituierte Derivate verwendet. Leider sind diese Stoffe in Wasser wenig löslich bzw. schwer dispergierbar. Dies führt leicht zu ungleichmäßigen oder zu schwachen Haarfärbungen. Insbesondere bei Färbezubereitungen mit hoher Farbstoffkonzentration kommt es leicht vor, daß Farbstoffe auskristallisieren und nicht auf das zu färbende Haar aufziehen. Es besteht daher ein dringendes Bedürfnis an direktziehenden Haarfarbstoffen mit verbesserter Wasserlöslichkeit.

Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Es wurde gefunden, daß die gestellten Anforderungen in hohem Maße erfüllt werden durch Haarfärbemittel mit einem Gehalt an direktziehenden Haarfarbstoffen in einem wäßrigen kosmetischen Träger, dadurch gekennzeichnet, daß als direktziehende Haarfarbstoffe eine oder mehrere Verbindungen der Formel (I)

(I)

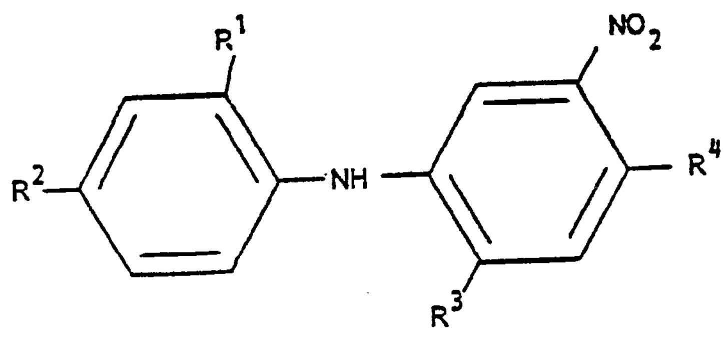

in der eine der Gruppen $R^1$ und $R^2$ eine Nitrogruppe und die andere eine $-SO_3H$ oder eine $-COOH$-Gruppe ist und eine der Gruppen $R^3$ und $R^4$ Wasserstoff und die andere eine Gruppe $-NR^5R^6$ ist, worin $R^5$ und $R^6$ Wasserstoff, Alkylgruppen mit 1 bis 4 C-Atomen oder Hydroxyalkylgruppen mit 2 bis 4 C-Atomen sind, oder

deren wasserlösliche Salze enthalten sind.

Unter den Verbindungen der Formel (I) sind wegen der leichten technischen Zugänglichkeit besonders solche bevorzugt, in welchen eine der Gruppen $R^3$ und $R^4$ Wasserstoff und die andere eine Aminogruppe oder eine 2-Hydroxyalkylaminogruppe ist.

Die Farbstoffe der Formel (I) erzeugen auf dem Haar orangegelbe bis braune Farbtöne von hoher Intensität, Licht- und Waschechtheit.

Die Dinitrophenyldiaminderivate der Formel (I) und deren wasserlösliche Salze sind neu und daher ein weiterer Gegenstand der Erfindung.

Die Herstellung der erfindungsgemäßen Verbindungen der Formel (I) erfolgt allgemein durch Umsetzung von 4-Chlor-3-nitro-benzoesäure, 4-Chlor-3-nitro-benzolsulfonsäure, 2-Chlor-5-nitrobenzoesäure oder 2-Chlor-5-nitrobenzolsulfonsäure mit einem Nitroanilin der Formel (II)

(II) 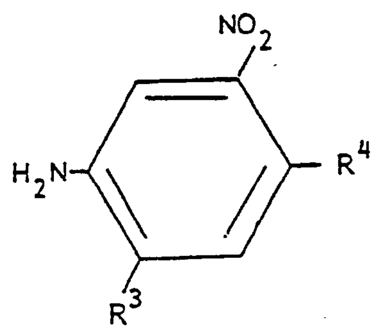

in welchem $R^3$ und $R^4$ die für Formel (I) genannte Bedeutung hat, unter Abspaltung von HCl in Gegenwart einer Base, z. B. eines Alkalicarbonats.

Unter den wasserlöslichen Salzen sind in erster Linie die Salze starker Basen wie etwa die Alkalisalze, z. B. das Natrium- oder Kaliumsalz oder Ammoniumsalze, Alkanolammoniumsalze mit 2 bis 4 C-Atomen in der Alkanolgruppe wie z. B. das Monoethanolammoniumsalz, das Triethanolammoniumsalz oder das Isopropanolammoniumsalz zu verstehen. Die erfindungsgemäßen Haarfärbemittel können die direktziehenden Nitrodiphenylaminderivate der allgemeinen Formel I allein oder in Kombination mit bekannten direktziehenden Farbstoffen, z.B. mit anderen Nitrobenzolderivaten, Anthrachinonfarbstoffen, Triphenylmethan- oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöle, Fettsäuren, ferner Parfümöle und haarpflegende Zusäzte, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das gesamte

Haarfärbemittel, eingesetzt. Daneben können bekannte Oxidationshaarfärbemittelvorprodukte (Entwickler- und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

### Herstellungsbeispiele

#### 1. 2,3'-Dinitro-4'-aminodiphenylamin-4-carbonsäure-Natriumsalz

Eine Mischung aus (A) 5,03 g (0,025 Mol) 4-Chlor-3-nitro-benzoesäure und (B) 3,82 g (0,025 Mol) 2-Nitro-p-phenylendiamin, 2,54 g (0,027 Mol) Natriumcarbonat und 10 ml Wasser wurde in einem Autoklaven 7 Stunden auf 120°C erhitzt. Nach dem Abkühlen wurde die Lösung zur Trockene eingeengt. Der Rückstand wurde aus einem Ethanol-Wasser-Gemisch umkristallisiert.

Braune Kristalle, Schmelzpunkt über 350°C (unter Zersetzung)

#### 2. 2,3'-Dinitro-4'-aminodiphenylamin-4-sulfonsäure-Natriumsalz

Die Herstellung erfolgte analog Beispiel 1 ausgehend von
(A) 4-Chlor-3-nitrobenzolsulfonsäure-Na-Salz und
(B) 2-Nitro-p-phenylendiamin
Braune Kristalle, Schmelzpunkt über 300°C

#### 3. 2,3'-Dinitro-4'-(2-hydroxyethyl)-aminodiphenylamin-4-carbonsäure

Herstellung analog Beispiel 1 ausgehend von
(A) 4-Chlor-3-nitrobenzoesäure und
(B) 4-(2-Hydroxyethyl)-amino-3-nitroanilin
Braune Kristalle, Schmelzpunkt über 238°C (unter Zersetzung)

#### 4. 2,3'-Dinitro-4-(2-hydroxyethyl)-aminodiphenylamin-4-sulfonsäure-Natriumsalz

Herstellung analog Beispiel 1 ausgehend von
(A) 4-Chlor-3-nitrobenzolsulfonsäure-Na-Salz und
(B) 4-(2-Hydroxyethyl)-amino-3-nitroanilin
Hellbraune Kristalle, Schmelzpunkt ab 280°C (unter Zersetzung)

#### 5. 3',4-Dinitro-4'-(2-hydroxyethyl)-aminophenylamin-2-carbonsäure-Natriumsalz

Herstellung analog Beispiel 1 ausgehend von
(A) 2-Chlor-5-nitrobenzoesäure und

(B) 4-(2-Hydroxyethyl)-amino-3-nitroanilin
Dunkelbraune Kristalle, Schmelzpunkt über 305°C

6. 3',4-Dinitro-6'-(2-hydroxyethyl)-aminodiphenylamin-2-carbonsäure

Herstellung analog Beispiel 1 ausgehend von
(A) 2-Chlor-5-nitrobenzoesäure und
(B) 2-(2-Hydroxyethyl)-amino-5-nitroanilin
Olivenbraune Kristalle, Schmelzpunkt ca. 132°C (unter Zersetzung)

7. 3',4-Dinitro-6'-aminodiphenylamin-2-carbonsäure

Herstellung analog Beispiel 1 ausgehend von
(A) 2-Chlor-5-nitrobenzoesäure und
(B) 4-Nitro-0-phenylendiamin
Orangebraune Kristalle, Schmelzpunkt 191 bis 196°C

8. 3',4-Dinitro-4'-aminodiphenylamin-2-carbonsäure

Herstellung analog Beispiel 1 ausgehend von
(A) 2-Chlor-5-nitrobenzoesäure und
(B) 2-Nitro-p-phenylendiamin
Braune Kristalle, Schmelzpunkt über 310°C

9. 3',4-Dinitro-4-(2-hydroxyethyl)-aminodiphenylamin-2-sulfonsäure-Natriumsalz

Herstellung analog Beispiel 1 ausgehend von
(A) 2-Chlor-5-nitrobenzolsulfonsäure-Na-Salz und
(B) 4-(2-Hydroxyethyl)-amino-3-nitroanilin
Braune Kristalle, Schmelzpunkt ca. 230°C (unter Zersetzung)

Haarfärbeversuche

Es wurden Haarfärbecremes der folgenden Zusammensetzung hergestellt :

| | |
|---|---|
| Fettalkohol $C_{12-18}$ | 10 g |
| Fettalkohol $C_{12-14}$ + 2 EO-sulfat, | |
| Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzen trierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90% ergrauten, aber nicht besonders vorbehandelten Menschenhaars aufgetragen und dort 30 Minuten bei 27°C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1 bis 9 eingesetzt.

Das Ergebnis der Färbeversuche ist der Tabelle 1 zu entnehmen.

Tabelle I

| direktziehender Farbstoff gemäß Beispiel | Nuance des gefärbten Haares |
|---|---|
| 1 | gelborange |
| 2 | mattgelb |
| 3 | beige |
| 4 | elfenbein |
| 5 | kamelbraun |
| 6 | braunorange |
| 7 | gelb |
| 8 | gelborange |
| 9 | goldblond |

**Patentansprüche**

1. Haarfärbemittel mit einem Gehalt an direktziehenden Haarfarbstoffen in einem wäßrigen kosmetischen Träger, dadurch gekennzeichnet, daß als direktziehende Haarfarbstoffe eine oder mehrere Verbindungen der Formel (I)

(I)

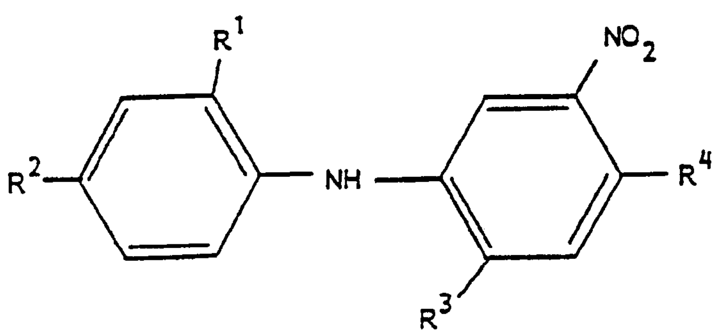

in der eine der Gruppen $R^1$ und $R^2$ eine Nitrogruppe und die andere eine —$SO_3H$ oder eine —COOH-Gruppe ist und eine der Gruppen $R^3$ und $R^4$ Wasserstoff und die andere eine Gruppe —$NR^5R^6$ ist, worin $R^5$ und $R^6$ Wasserstoff, Alkylgruppen mit 1 bis 4 C-Atomen oder Hydroxyalkylgruppen mit 2 bis 4 C-Atomen sind, oder deren wasserlösliche Salze enthalten sind.

2. Haarfärbemittel nach Anspruch 1, dadurch gekennzeichnet, daß eine der Gruppen $R^3$ und $R^4$ Wasserstoff und die andere eine Aminogruppe oder eine 2-Hydroxyethylaminogruppe ist.

3. Haarfärbemittel nach den Ansprüchen 1 oder 2, dadurch gekennzeichnet, daß die direktziehenden Haarfarbstoffe der Formel (I) in einer Menge von 0,01 bis 5 Gew.-%, bezogen auf das gesamte Haarfärbemittel, enthalten sind.

4. Haarfärbemittel nach den Ansprüchen 1 bis 3, dadurch gekennzeichnet, daß zusätzlich zu den Verbindungen der Formel (I) noch weitere direktziehende Haarfarbstoffe und/oder Oxidationsfarbstoffvorprodukte enthalten sind und daß diese Farbstoffe in einem kosmetischen Träger in Form einer Creme, einer Emulsion, eines Gels, eines Shampoos oder eines Schaumaerosols eingearbeitet sind.

5. Dinitro-diphenylaminderivate der Formel (I)

(I)

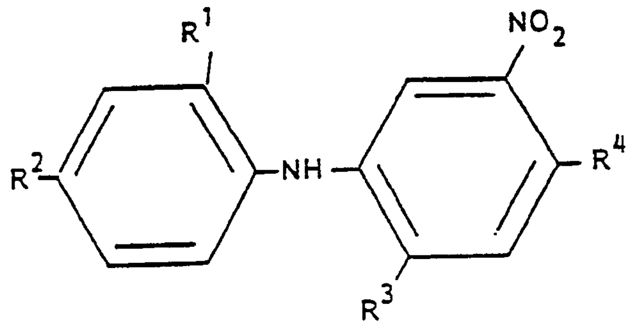

in der eine der Gruppen $R^1$ und $R^2$ eine Nitrogruppe und die andere eine —$SO_3H$ oder eine —COOH-Gruppe ist und eine der Gruppen $R^3$ und $R^4$ Wasserstoff und die andere eine Gruppe —$NR^5R^6$ ist, worin $R^5$ und $R^6$ Wasserstoff, Alkylgruppen mit 1 bis 4 C-Atomen oder Hydroxyalkylgruppen mit 2 bis 4 C-Atomen sind oder deren wasserlösliche Salze.

## Claims

1. Hair-dyeing preparations containing substantive hair dyes in an aqueous cosmetic carrier, characterized in that they contain as substantive hair dyes one or more compounds corresponding to the following formula

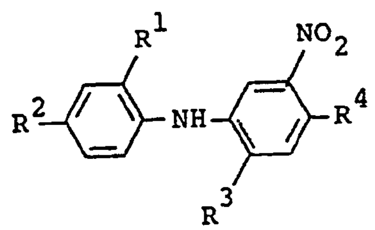

(I)

in which one of the groups $R^1$ and $R^2$ is a nitro group while the other is an —$SO_3H$ or a —COOH group and one of the groups $R^3$ and $R^4$ is hydrogen while the other is a group of the formula —$NR^5R^6$, where $R^5$ and $R^6$ are hydrogen, $C_1$-$C_4$ alkyl groups or $C_2$-$C_4$ hydroxyalkyl groups, or water-soluble salts of these compounds.

2. Hair-dyeing preparations as claimed in claim 1, characterized in that one of the groups $R^3$ and $R^4$ is hydrogen while the other is an amino group or a 2-hydroxyethylamino group.

3. Hair-dyeing preparations as claimed in claim 1 or 2, characterized in that the substantive hair dyes of formula (I) are present in a quantity of 0.01 to 5% by weight, based on the hair-dyeing preparation as a whole.

4. Hair-dyeing preparations as claimed in claims 1 to 3, characterized in that they contain other substantive hair dyes and/or oxidation dye precursors in addition to the compounds of formula (I) and in that these dyes are incorporated in a cosmetic carrier in the form of a cream, an emulsion, a gel, a shampoo or a foam aerosol.

5. Dinitrodiphenylamine derivatives corresponding to the following formula

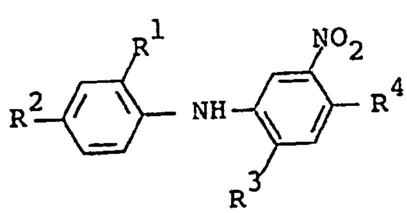

(I)

in which one of the groups $R^1$ and $R^2$ is a nitro group while the other is an —$SO_3H$ or a —COOH group and one of the groups $R^3$ and $R^4$ is hydrogen while the other is a group of the formula —$NR^5R^6$, where $R^5$ and $R^6$ are hydrogen, $C_1$-$C_4$ alkyl groups or $C_2$-$C_4$ hydroxyalkyl groups, or water-soluble salts thereof.

**Revendications**

1. Produit colorant pour cheveux ayant une teneur en colorants pour cheveux montant directement sur la fibre dans un support cosmétique aqueux, caractérisé en ce l'on utilise comme colorant pour cheveux montant directement sur la fibre un ou plusieurs composés de la formule (I)

(I) 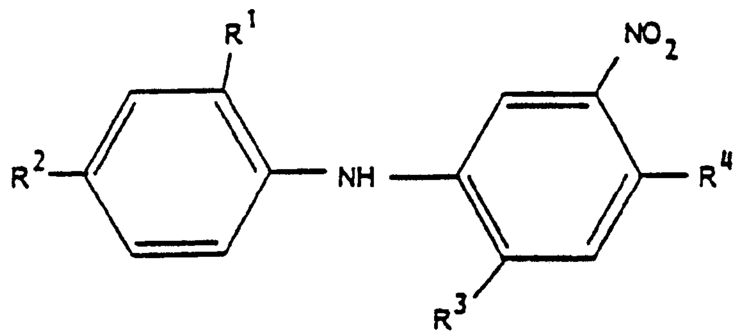

dans laquelle l'un des groupes $R^1$ et $R^2$ représente un groupe nitro et l'autre un groupe —$SO_3H$ ou —COOH et dans laquelle l'un des groupes $R^3$ et $R^4$ est de l'hydrogène et l'autre un groupe —$NR^5R^6$, où $R^5$ et $R^6$ sont de l'hydrogène, des groupes alkyle à 1 à 4 atomes de carbone et des groupes hydroxyalkyle à 2 à 4 atomes de carbone ou leurs sels solubles dans l'eau.

2. Produit colorant pour cheveux selon la revendication 1, caractérisé en ce que l'un des groupes $R^3$ et $R^4$ est de l'hydrogène et l'autre est un groupe amino ou un groupe 2-hydroxyéthylamino.

3. Produit colorant pour cheveux selon la revendication 1 ou 2, caractérisé en ce que les colorants pour cheveux montant directement sur la fibre de la formule (I) sont présents en quantités de 0,01 à 5% en poids rapportées au total du produit colorant pour cheveux.

4. Produit colorant pour cheveux selon les revendications 1 à 3, caractérisé en ce que, outre les composés de la formule (I), il existe encore d'autres colorants pour cheveux montant directement sur la fibre et/ou des précurseurs d'oxydation pour colorants,et que ces colorants sont incorporés à un support cosmétique sous forme d'une crème, d'une émulsion, d'un gel, d'un shampooing ou d'une mousse en aérosol.

5. Dérivé de la dinitro-diphenylamine de la formule (1)

(I) 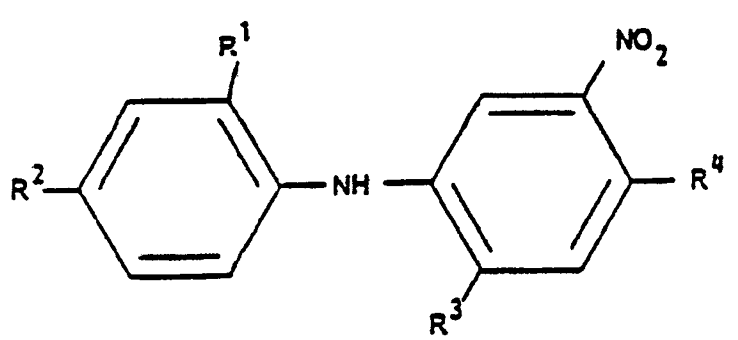

dans laquelle l'un des groupes $R^1$ et $R^2$ représente un groupe nitro et l'autre un groupe —$SO_3H$ ou —COOH et dans laquelle l'un des groupes $R^3$ et $R^4$ est de l'hydrogène et l'autre est un groupe —$NR^5R^6$, où $R^5$ et $R^6$ sont de l'hydrogène, des groupes alkyle à 1 à 4 atomes de carbone ou des groupes hydroxyalkyle à 2 à 4 atomes de carbone ou leurs sels solubles dans l'eau.